# FASCICULE DE BREVET EUROPEEN

(11) **EP 1 993 716 B1**
(45) Date de publication et mention de la délivrance du brevet: **31.07.2019**
(21) Numéro de dépôt: 07731154.6
(22) Date de dépôt: 16.03.2007
(51) Int. Cl.: B01J 2/02, B01J 2/30, B01D 1/18, C07H 3/04, C13K 13/00, A61K 31/7016

(54) **PROCÉDÉ DE PRÉPARATION D'UNE COMPOSITION PULVÉRULENTE**
VERFAHREN ZUR HERSTELLUNG EINER PULVERFÖRMIGEN ZUSAMMENSETZUNG
METHOD OF PREPARING A PULVERULENT COMPOSITION

(30) Priorité: 17.03.2006 FR 0602383
(43) Date de publication de la demande: 26.11.2008
(73) Titulaire: Innov'ia, 17000 La Rochelle (FR)
(72) Inventeur: BUISSON, Pierre, 17140 Lagord (FR); CHESSE, Claude, 17000 La Rochelle (FR)
(74) Mandataire: Grosset-Fournier, Chantal Catherine
(86) Numéro de dépôt international: PCT/FR2007/000462
(87) Numéro de publication internationale: WO 2007/118949

(56) Documents cités:
- EP-A1- 0 945 173
- FR-A1- 2 838 654
- US-A- 4 142 916
- US-A- 5 307 640
- US-A- 5 415 695
- US-A- 6 063 138

## Description

La présente invention a pour objet un procédé de préparation d'une composition pulvérulente de lactulose.

Les procédés de séchage par atomisation sont bien connus de l'homme du métier. De manière générale, une dispersion aqueuse d'une substance qui doit être séchée est pulvérisée dans un courant d'air chaud passant dans une chambre de séchage avec récupération des produits secs obtenus sous forme de poudre. Le séchage par atomisation est utilisé dans l'industrie alimentaire pour le séchage de produits tels que le lait, le café, les préparations chocolatées, les jus de fruits, les extraits végétaux et animaux, les produits issus de fermentation et d'une manière générale de nombreux ingrédients et additifs destinés à des applications alimentaires, cosmétiques, pharmaceutiques ou de la chimie fine. Les procédés de séchage par atomisation sont nombreux et sont particulièrement bien décrits dans le site niro®.com, notamment en ce qui concerne le séchage par atomisation tour simple effet, le séchage "deux temps" par tour à fond W, le séchage par tour multiple effet et le séchage type Filtermat® avec tour d'atomisation intégrant un sécheur à bande.

Pourtant, il est reconnu par l'homme du métier qu'il est difficile voire impossible d'utiliser la technique de séchage par atomisation, y compris en associant des étapes complémentaires de séchage par fluidisation, pour le séchage de certains matériaux, tels que certains sucres par exemple et en particulier si la pureté de ce sucre est réduite par la présence d'autres molécules de sucres ou par d'autres composants. Tel est le cas du lactulose dont les sirops industriels présentent des teneurs en lactulose comprises entre 50% et 100% et de préférence entre 60 et 98% ; ainsi, lorsqu'une solution de lactulose est séchée par un procédé de séchage par atomisation traditionnel, il se forme très rapidement un verre amorphe incorporant l'eau. Les particules de verre de lactulose collent progressivement sur les parois de la tour et le procédé s'arrête de lui-même, compte tenu des phénomènes de collage engendrés. Ce caractère d'hygroscopicité se rencontre pour une large part dans les hydrates de carbone (lactose, glucose, sucrose, fructose, sorbose, tagatose, xylose et ce sans limitation, et du lactulose par exemple), et rendent les produits non conformes pour de nombreuses applications commerciales. D'autres matières incluant en particulier des acides organiques (acide citrique, acide tartrique, acide malique, acide lactique et sans limitations), certains dérivés de fermentation (extraits de levures par exemple), des substances protéiques à faible poids moléculaires (peptides, acides aminés) et les gommes naturelles ou issues de fermentation peuvent prendre de la même façon des formes de verre lorsqu'elles sont séchées par atomisation. Ces formes hygroscopiques ne sont pas directement compressibles.

Les produits réputés hygroscopiques comme le lactulose présentent de nombreuses applications dans des domaines variés. Par exemple, le lactulose est bien connu pour son efficacité concernant le traitement de la constipation et de l'encéphalopathie hépatique, mais aussi pour ses propriétés prébiotiques, c'est-à-dire d'activateur de croissance de micro-organismes bifidogènes. Ainsi, il est connu que le lactulose rajouté dans une poudre de lait infantile favorise la production de L. Bifidus dans la flore intestinale du nourrisson, de manière semblable à ce qui existe lorsque le nourrisson est alimenté au lait maternel. Ceci démontre que l'utilisation du lactulose, tant dans le domaine pharmaceutique humain ou vétérinaire que comme additif alimentaire à caractère nutritionnel, est recherché, et que le champ des applications est très large.

En raison de son caractère hygroscopique reconnu, le lactulose reste aujourd'hui utilisé principalement sous la forme d'un sirop dont la concentration sur extrait sec est variable de 50% à 100%.

De nombreux procédés de l'état de la technique ont été recherchés dans le but d'obtenir du lactulose sous forme d'une poudre stable.

Ainsi, le brevet US 5,326,405 décrit une méthode de préparation de lactulose cristallisé en pratiquant de manière simultanée l'agitation et le chauffage d'une solution de lactulose pour évaporer l'eau tout en incorporant des cristaux jusqu'à l'obtention d'une poudre fluide. L'inconvénient de cette technique réside dans le caractère discontinu de la méthode et la difficulté de transférer le procédé à l'échelle industrielle dans des conditions économiques acceptables. Il est par ailleurs nécessaire de disposer de cristaux pour l'ensemencement et ces cristaux, avec les risques de contamination induits et l'introduction d'une étape complémentaire et coûteuse.

Le brevet US 5,415,695 décrit un procédé de préparation de formes sèches par évaporation d'un sirop de lactulose pour réduire la teneur en eau suivie d'une opération de refroidissement jusqu'à solidification. Le produit solide peut être broyé. La méthode nécessite de procéder à un refroidissement rapide de la solution de lactulose. L'inconvénient, outre l'aspect de consommation énergétique importante du procédé, est d'introduire une opération complémentaire de broyage et tamisage coûteuse et entraînant la formation de fines "poussières", avec les risques de pertes de produit, donc de baisse de rendement. La présence de fines peut par ailleurs augmenter les risques de contamination croisée lors de la mise en oeuvre des produits, et accroître les problèmes de collage sur les parois de reprise en masse au stockage.

Le document EP 0 622 374 décrit un procédé voisin conduisant à la formation d'un trihydrate lactulose cristallisé avec les inconvénients énumérés de la complexité et du coût de tels procédés, limitant de manière importante le champ d'applications, notamment pour les applications alimentaires.

La demande internationale WO 98/19684 décrit une méthode de séchage par pulvérisation d'une solution de lactulose à contre courant sur un lit fluidisé ; toutefois pour obtenir la sortie du produit sec il est nécessaire de rajouter un agent d'absorption ou de gélification pour absorber l'eau. L'inconvénient réside encore une fois sur le caractère discontinu du procédé et l'obligation de procéder à l'addition de substances étrangères.

La demande internationale WO 00/36153 décrit une méthode de séchage d'une solution de lactulose en mettant en oeuvre un procédé de séchage sous vide, la solution étant chauffée à haute température sous vide : la production de mousse permet de favoriser le séchage et d'obtenir un gâteau sec qui est ensuite broyé pour l'obtention d'une poudre de lactulose. Le procédé présente l'inconvénient de travailler à haute température avec les risques de brunissement éventuel lié aux réactions de Maillard, si la solution de lactulose n'est pas suffisamment purifiée et surtout l'introduction d'une opération supplémentaire de broyage tamisage, avec risque de pertes de matières, production de fines et les inconvénients déjà décrits.

Il est important de souligner que les poudres issues de sirops industriels de lactulose présentent une température de transition vitreuse beaucoup plus faible, et d'autant plus que la poudre n'est pas anhydre ; ainsi à une teneur en eau de 3%, les poudres de lactulose issues de sirops industriels présentent une température de transition vitreuse comprise de 35°C à 75°C, ce qui entraîne des reprises rapides d'humidité et des problèmes de collage.

Dans Paper number 046004 ASAE Annual meeting 2004, on décrit les corrélations entre transition vitreuse et température de point de collage de poudres alimentaires réputées difficilement séchables par atomisation voire inséchables sans support d'atomisation ; elles présentent des température de transition vitreuse (Tg) faibles, et sont très hygroscopiques dans leur état amorphe. Lors du séchage par atomisation pour lequel les temps de résidence sont très courts, la température de transition vitreuse diminue et on obtient la transformation de produits solubles tels que sucres et acides organiques par exemple sous leur forme amorphe. En présence d'un air de séchage non déshumidifié, l'eau joue le rôle de plastifiant et abaisse progressivement la température de transition vitreuse avec l'augmentation de l'humidité et de l'activité d'eau, entraînant des phénomènes de collage non contrôlés dans les installations de séchage. Pour remédier à ce problème, l'homme du métier utilise des supports d'atomisation qui présentent des températures de transition vitreuse élevées, tels que des isolats protéiques, des maltodextrines, qui sont rajoutés à la solution à sécher. La relation entre température de transition vitreuse et activité d'eau permet de prédire la stabilité des poudres obtenues au stockage.

De par ses propriétés hygroscopiques, de nombreuses tentatives ont été recherchées pour résoudre ces problèmes, notamment pour le séchage par atomisation de solution à plus ou moins haute pureté en lactulose et à plus ou moins haute teneur en matière sèche.

Ainsi, les brevets NE129368, 147784 et 150161 décrivent des méthodes de préparation de formes sèches à base de lactulose obtenues par atomisation nécessitant dans tous les cas l'adjonction de supports d'atomisation (farine de riz) ; les inconvénients de ces procédés résident d'une part dans la diminution de la concentration en lactulose en poids sur poids dans la préparation pulvérulente, et dans l'introduction de substances nouvelles entraînant un surcoût et qui se retrouveront dans les sirops lors de la remise en solution par exemple, avec les contraintes réglementaires attachées.

Le brevet US 3,716,408 décrit une poudre de lactulose à 55% de teneur en lactulose obtenu par atomisation. Pour remédier au grave inconvénient de poudre hygroscopique, il est nécessaire d'incorporer un agent extérieur, plus particulièrement une poudre de Konjac dans la solution dans le but de permettre le séchage du mélange ; le procédé présente l'inconvénient de l'addition d'un adjuvant, qui peut être interdit pour certaines applications ou/et réglementations et dont il conviendra obligatoirement de faire mention sur l'étiquetage tant dans le cas d'une application pharmaceutique qu'alimentaire.

Le brevet JP778565 décrit un procédé de séchage par atomisation pour obtenir une poudre à 55% de concentration de lactulose, mais dans laquelle on rajoute une protéine pour servir d'adjuvant de séchage ; cette poudre présente également des caractères d'instabilité notoire en ambiance humide avec l'inconvénient d'un adjuvant complémentaire.

US 4 142 916 A décrit la préparation des compositions pulvérulentes de lactulose non hygroscopiques.

La présente invention a pour but de fournir un procédé de préparation d'une composition pulvérulente stable et non hygroscopique à partir d'un produit initialement hygroscopique. Plus particulièrement, la présente invention a pour but de fournir un procédé de préparation d'une poudre de lactulose non hygroscopique et stable.

La présente invention concerne un procédé de préparation d'une composition pulvérulente non hygroscopique, comprenant une étape de séchage par atomisation, sans support d'atomisation, d'une solution aqueuse contenant au moins un produit initialement hygroscopique, par exemple sous forme liquide, présentant une température de transition vitreuse de 10°C à 110°C, et un fluide cryogénique, notamment un fluide cryogénique alimentaire, ou un mélange de fluides cryogéniques, notamment choisi parmi l'anhydride carbonique, l'azote, l'air liquide, ladite solution aqueuse étant obtenue par dissolution dudit fluide cryogénique dans une solution aqueuse initiale contenant ledit produit initialement hygroscopique. Le produit initialement hygroscopique est le lactulose.

Le procédé de la présente invention est caractérisé en ce qu'il ne comprend pas l'utilisation d'un adjuvant de séchage.

L'expression "séchage par atomisation" désigne un procédé de séchage d'un liquide en pulvérisant ledit liquide dans un courant d'air chaud par des buses ou des turbines. Le séchage et le transfert de l'eau se font par entraînement dans l'air du fait de la différence de pression de vapeur entre la gouttelette formée et l'air à la périphérie de la gouttelette.

L'expression "support d'atomisation" désigne une substance présente sous sa forme sèche non hygroscopique telle que maltodextrine, amidon modifié, fibres, gommes, et protéines, rajoutée dans des proportions de 2% à 75% (poids sec) dans une solution aqueuse contenant au moins un produit initialement hygroscopique permettant de diminuer le caractère collant et hygroscopique de la poudre.

L'hygroscopicité d'un produit se définit comme l'affinité dudit produit pour l'eau. Cette affinité pour l'eau a une influence sur les isothermes de sorption et donc sur l'activité d'eau (a_{w}) et la teneur en eau. Ces quatre facteurs (a_{w}, teneur en eau, composition et hygroscopicité) sont interdépendants. L'hygroscopicité est responsable de l'adsorption de la vapeur d'eau ambiante par ledit produit, en opposition avec la désorption. Les poudres peuvent être classées en cinq catégories selon leur hygroscopicité (Séchage des lactosérums et dérivés, Rôle du lactose et de la dynamique de l'eau, Pierre Schuck et al., Lait, 84 (2004) 243-268) :

| Classes | Pourcentage d'hygroscopicité ou teneur en eau finale après exposition à un air humide à 80% d'humidité résiduelle |
|---|---|
| Non hygroscopique | ≤ 10,0 |
| Peu hygroscopique | 10,1-15,0 |
| Moyennement hygroscopique | 15,1-20,0 |
| Hygroscopique | 20,1-25,0 |
| Très hygroscopique | > 25,0 |

Comme produits hygroscopiques, on peut citer le galactose dont la température de transition vitreuse est égale à 32°C et l'acide tartrique dont la température de transition vitreuse est égale à 18°C.

Le lactulose quant à lui présente une température de transition vitreuse de 94°C.

D'autres produits hygroscopiques sont présentés dans le tableau qui suit :

| Sucres | Hygroscopicité relative | Température de fusion (°C) | Solubilité dans l'eau à 60°C (%poids/poids) | Température de transition vitreuse (°C) | Propriété collante (relative) |
|---|---|---|---|---|---|
| Lactose | + | 223 | 35 | 101 | + |
| Maltose | ++ | 165 | 52 | 87 | ++ |
| Sucrose | +++ | 186 | 71 | 62 | +++ |
| Glucose | ++++ | 146 | 72 | 31 | +++++ |
| Fructose | ++++++ | 105 | 89 | 5 | ++++++ |

Le fluide cryogénique peut être utilisé pour l'étape de dissolution sous forme liquide ou gazeuse. De préférence, on utilise un fluide cryogénique alimentaire.

La présente invention permet de produire une préparation pulvérulente de lactulose dont la pureté est comprise de 50% à 100%, à très haute concentration, avec des performances améliorées par rapport à celles des préparations pulvérulentes de lactulose de l'art antérieur, permettant d'obtenir des poudres aux propriétés physiques nouvelles et surprenantes : elles présentent une très grande stabilité de conservation à l'air libre sans reprise en masse, démontrant l'absence du caractère d'hygroscopicité, contrairement aux poudres de lactulose de l'art antérieur. Le simple toucher d'une poudre hygroscopique de lactulose laisse une empreinte immédiate sur le doigt liée à l'adhérence instantanée provoquée par le réchauffement de la poudre en contact avec le doigt. La présente invention permet de produire une poudre qui dispose d'une inertie de plusieurs dizaines de secondes à la température de peau, ce qui est un avantage très substantiel dans le cas de manipulations de sachets de poudre de lactulose par exemple.

La présente invention permet également de fournir une poudre de lactulose stable sans adjonction de supports d'atomisation dans la solution, à partir d'une solution de lactulose pouvant présenter un degré de pureté variable, compris entre 50% et 98%, pour les cas les plus courants et les plus difficiles, mais aussi pour des puretés supérieures à 98% ou inférieures à 50%.

La présente invention permet également d'obtenir, de manière surprenante, des poudres de lactulose parfaitement blanches, apportant un avantage supplémentaire de neutralité complète de l'influence de la coloration, lors de la mise en oeuvre de nouvelles préparations sèches à base de lactulose ; cette propriété est à comparer avec la couleur beige des poudres de l'état de la technique.

La présente invention permet également de fournir une poudre de lactulose à haute concentration présentant d'excellentes propriétés de mouillabilité et de vitesse de solubilisation instantanée.

La présente invention permet également de fournir une poudre de lactulose présentant un minimum de fines, cette absence de poussières évitant tous les risques de contamination aéroportée lors de la remise en oeuvre du produit, et diminue les risques éventuels d'allergies par inhalation de poussières du produit.

La présente invention permet également de fournir une poudre de lactulose dont le degré de pureté variable peut être compris entre 50% et 98% pour les cas les plus courants et les plus difficiles, mais aussi pour des puretés supérieures à 98% ou inférieures à 50%, directement compressibles, possédant des propriétés d'écoulement favorables à la compression.

L'homme du métier connaît les travaux réalisés permettant d'expliquer les phénomènes de collage de poudre liées pour l'essentiel aux caractères d'hygroscopicité et de themoplasticité des poudres ; il est connu qu'à une température comprise entre 75°C et 100°C la poudre de lactulose présente une très grande thermoplasticité.

La transition vitreuse est un changement d'état d'une substance sous l'effet de la température, entraînant des variations importantes de ses propriétés mécaniques. La transition vitreuse se caractérise par la température de transition : au dessus de cette température le produit présente une structure plastique (état viscoélastique) ; au dessous de cette température le produit présente une structure dite vitreuse (état solide) et présente le comportement d'un produit solide élastique.

La température de transition vitreuse du lactulose anhydre pur est de 90°C à 95°C ; celle-ci s'abaisse très rapidement de 10°C à 40°C en fonction de la teneur en eau de la poudre en équilibre avec l'air et ce pour des tensions de vapeur de cet air proche de 5% ; elle baisse également avec la pureté et décrit ainsi une plage de température dans laquelle le produit présente des propriétés de thermoplasticité (voir « Notion de transition vitreuse appliquée au séchage par pulvérisation de solutions glucidiques », Laurence Busin, Pierre Buisson, Jacques Bimbenet, Sciences de l'aliment 16 (1996) 443-459).

Ce modèle ne décrit pas la cinétique du phénomène de transfert permanent de chaleur et d'eau ; si pour le lactulose anhydre pur la température de transition vitreuse est de 90°C à 95°C, la température de transition vitreuse d'une solution de lactulose technique obtenue industriellement, contenant en outre du lactulose d'autres sucres (lactose, galactose, fructose, etc.) est nécessairement plus basse.

De manière surprenante, il a été constaté qu'il existait pour une humidité inférieure à 2%, une zone préférentielle de stabilisation de la poudre de lactulose comprise d'environ 50°C à environ 85°C, de préférence d'environ 65°C à environ 80°C, et notamment d'environ 65°C à environ 75°C, conduisant à l'élimination de tous les phénomènes de collage dans les cyclones de la tour d'atomisation, en maîtrisant la pulvérisation de la solution dans un courant d'air chaud à des conditions d'enthalpies maîtrisées.

De manière inattendue et très surprenante, il a été constaté un brusque changement d'état définitivement stable de particules de poudre de lactulose à forte thermoplasticité, lorsque les particules étaient soumises à un sous-refroidissement, par la détente rapide d'un fluide cryogénique alimentaire tel que par exemple l'anhydride carbonique ou l'azote ou l'air liquide et de préférence l'anhydride carbonique injecté à une pression comprise entre 10⁵ Pa et 5 × 10⁶ Pa, et de préférence entre 5x 10⁵ Pa et 10 × 10⁵ Pa, l'anhydride carbonique se présentant pour une part sous la forme liquide dans la solution et pour l'autre part sous la forme gazeuse. Toutefois, le phénomène est d'autant plus amélioré par la présence des molécules d'anhydride carbonique dissous dans la solution. En effet, lors de la détente du fluide cryogénique dissous lors de la pulvérisation, les frigories sont apportées par la chaleur latente de changement d'état, soit 64 frigories par kg d'anhydride carbonique liquide (38 frigories par kg pour l'azote), et par la chaleur sensible du gaz anhydride carbonique conduisant à disposer d'environ 100 frigories par kilogramme de gaz anhydride carbonique. Cette injection permet la stabilisation de la poudre dans la tour de séchage dans des zones de température de 20°C à 100°C.

Ainsi, la présente invention permet également d'obtenir une poudre de lactulose, en procédant au surséchage en continu de la poudre obtenue en la soumettant à un sous-refroidissement « in situ » instantané par la maîtrise de barèmes de températures basses comprises entre 20°C et 100°C, et de préférence entre 40°C et 75°C.

La présente invention permet également d'obtenir une poudre de lactulose dans les conditions décrites ci-dessus, tout en soumettant les particules de lactulose à une détente rapide d'un fluide frigorigène de qualité alimentaire (anhydride carbonique ou azote par exemple, et de préférence l'anhydride carbonique) qui n'apporte pas d'humidité complémentaire, ce qui dans le cas contraire, conduirait à une réversibilité du changement d'état et un retour à un état thermoplastique instable.

L'un des autres aspects de la présente invention est aussi de procéder, comme dans les conditions décrites ci-dessus, en injectant du CO₂ sous pression à l'état de gaz ou de liquide (gaz carbonique ou azote par exemple sans que ceci représente de caractère limitatif) dans la solution préchauffée, permettant de manière surprenante d'autoriser la pulvérisation de la solution chauffée carbonatée à des concentrations de solutions très élevées et présentant des viscosités critiques très élevées jusqu'à 2000 centipoises, alors que les viscosités critiques habituellement connues et utilisées par l'homme de l'art lors du séchage par pulvérisation dans un courant d'air chaud, se situent entre 50 à 200 centipoises. Ce taux de concentration supérieur présente le double avantage de réduire d'une part, l'humidité de l'air d'équilibre de séchage, c'est à dire la quantité d'eau dans l'air rapportée lors du transfert adiabatique et d'autre part d'augmenter très largement la productivité et donc l'intérêt économique du procédé.

Ainsi, alors que tous les tests et expériences conduits tant sur des tours de séchage par atomisation à turbine simple effet (Tour NIRO Minor mobile®) que sur tour de séchage multiple effet (Tour NIRO MSD 20®) équipée soit de buse mono haute pression, soit de buse bi-fluide ou que sur tour de séchage à deux effets fond W à buse ont conduit à un constat de collage rapide sans possibilité de fonctionnement dans un régime stabilisé, la présente invention a démontré qu'il était possible d'obtenir sur chacune de ces tours de séchage par atomisation et configuration de tour de séchage par atomisation : tour simple effet, tour dite multiple effet équipée soit de buse mono haute pression, soit de buse bi-fluide, tour de séchage par atomisation à deux effets fond W à buse, tour dite « à cigare » ou tall form, tour d'atomisation équipée d'un sécheur à bandes intégré en bas de tour de type Filtermat®, à une échelle industrielle, et dans des conditions économiques répondant aux exigences du marché, une poudre de lactulose stable dont la pureté en lactulose peut être comprise entre 50% et 100% exprimée en poids de lactulose dont l'hygroscopicité est fortement réduite, sans utiliser des procédés industriels coûteux, ni procéder à un ajout de support de séchage ou de support d'atomisation (facteur de dilution) ou sans devoir procéder à une purification coûteuse des solutions de lactulose mises en oeuvre. L'invention concerne aussi le procédé de séchage par atomisation d'une solution de lactulose technique liquide concentrée et de viscosité importante pouvant être comprise entre 50 et 2000 centipoises, solution préalablement chauffée pendant environ 1 à environ 10 minutes, de préférence d'environ 2 à environ 5 minutes, à une température d'environ 20°C à environ 75°C, et dans laquelle a été dissous sous pression sous forme liquide ou de gaz un fluide cryogénique, de préférence de l'anhydride carbonique. La pulvérisation de la solution chaude et carbonatée, dans le cadre de l'utilisation d'anhydride carbonique, est effectuée de manière concomitante avec un agent anti-agglomérant choisi dans la gamme des anti-agglomérants connus de l'homme de l'art, et de préférence de type silice colloïdale.

Selon un mode de réalisation avantageux, le procédé de préparation de l'invention est caractérisé en ce que l'étape de séchage par atomisation s'accompagne d'une stabilisation primaire résultant du refroidissement de la composition pulvérulente obtenue pendant l'étape de séchage par atomisation, lequel refroidissement est provoqué par la détente du fluide cryogénique dissous dans la solution aqueuse initiale.

Selon un autre mode de réalisation avantageux, le procédé de préparation de l'invention est caractérisé en ce que le refroidissement de la composition pulvérulente obtenue pendant l'étape de séchage par atomisation a lieu dans une gamme de température inférieure à la température de transition vitreuse du produit hygroscopique, et en ce que la teneur en eau de ladite composition pulvérulente obtenue à l'issue de la susdite étape est inférieure à environ 7%, et est notamment d'environ 1% à environ 4%.

Parmi les produits hygroscopiques, on peut citer des produits organiques dont la masse moléculaire moyenne est inférieure à environ 1 000 Da, notamment comprenant au moins 50% en poids de glucides, tels que le lactulose, les fructo-oligosaccharides, les galacto-oligosaccharides, le fructose, le maltose, le lactose, le saccharose, le glucose, l'inuline ou des mélanges de ceux-ci, des polyols tels que le sorbitol, le maltitol ou le xylitol, des compositions à base de miel, des produits dérivés de l'extraction du lactose, le lactosérum ou ses dérivés, des mélanges de sucres et édulcorants, tels que des mélanges de fructo-oligosaccharides et d'aspartame, d'acésulfame ou de rhamnose.

Parmi les produits hygroscopiques, on peut également citer les extraits de plantes hygroscopiques tels que du sérum de luzerne obtenu après extraction des protéines, le contenu cytoplasmique de cellules de plantes fraîches, des extraits d'artichaut ou des extraits polyphénoliques végétaux (notamment raisin ou pomme), les mélanges associant un ou plusieurs probiotiques (et notamment ceux de la famille des Lactobacillus et des Bacillus), et un ou plusieurs actifs d'intérêt nutritionnel et notamment les prébiotiques (notamment le lactulose, les fructo-oligossacharides, le rhamnose), les acides gras poly-insaturés (tels que des extraits d'huile de poissons riches en ω3), les polyphénols (notamment les catéchols et les extraits de pépin de raisin), et les extraits de levure.

La présente invention concerne un procédé de préparation d'une composition pulvérulente de lactulose comprenant une étape de séchage par atomisation d'une solution aqueuse de lactulose et un fluide cryogénique alimentaire, notamment choisi parmi l'anhydride carbonique, l'azote, l'air liquide ou un mélange de ceux-ci, ladite solution aqueuse étant obtenue par dissolution dudit fluide cryogénique alimentaire dans une solution aqueuse initiale contenant le lactulose.

La présente invention concerne un procédé de préparation tel que défini ci-dessus, dans lequel l'étape de séchage par atomisation est précédée d'une étape de pulvérisation simultanée d'un agent anti-agglomérant et de la solution aqueuse contenant le produit initialement hygroscopique, le lactulose, et le fluide cryogénique alimentaire.

L'expression "agent anti-agglomérant" désigne une substance, généralement sous forme de poudre, absorbant l'eau ajoutée aux aliments pour empêcher leur agglomération ou maintenir leur fluidité.

Dans le cadre de la présente invention, l'agent anti-agglomérant ne joue pas le rôle de support d'atomisation.

Selon un mode de réalisation avantageux, le procédé de l'invention est caractérisé en ce que la concentration d'agent anti-agglomérant est inférieure à environ 0,5%, et varie de préférence d'environ 0,1% à environ 0,3% en poids d'extrait sec de l'anti-agglomérant par rapport au poids d'extrait sec de la composition pulvérulente non hygroscopique, de lactulose.

Selon un mode de réalisation avantageux, le procédé de l'invention est caractérisé en ce que l'agent anti-agglomérant est choisi parmi : la silice colloïdale, les silicates, le carbonate de magnésium, le calcium, le talc et le phosphate.

Selon un mode de réalisation préféré, la présente invention concerne un procédé de préparation tel que défini ci-dessus, caractérisé en ce que l'étape de séchage par atomisation est effectuée avec de l'air chaud à une température d'environ 100°C à environ 250°C, de préférence d'environ 115°C à environ 150°C.

La présente invention concerne également un procédé de préparation tel que défini ci-dessus, caractérisé en ce que la solution aqueuse initiale contenant le produit initialement hygroscopique, notamment le lactulose, est à une température d'environ 50°C à environ 85°C, de préférence d'environ 65°C à environ 80°C.

Un procédé de préparation préféré selon la présente invention est caractérisé en ce que la solution aqueuse initiale contenant le produit initialement hygroscopique, notamment le lactulose, présente une concentration en matières sèches d'environ 20% à environ 80% en poids de matière sèches par rapport au poids de la solution aqueuse initiale, et de préférence d'environ 60% à environ 70%.

La présente invention concerne également un procédé de préparation tel que défini ci-dessus, caractérisé en ce que la solution aqueuse initiale contenant le produit initialement hygroscopique, notamment le lactulose, contient d'environ 20% à environ 100%, notamment d'environ 50% à environ 100%, et de préférence d'environ 60% à environ 80%, en poids de produit hygroscopique par rapport à la totalité du poids de matières sèches.

Selon un mode de réalisation préféré, le fluide cryogénique est à une pression comprise d'environ 10⁵ Pa à environ 20 × 10⁵ Pa, de préférence d'environ 4 × 10⁵ Pa à environ 12 × 10⁵ Pa.

Un procédé de préparation particulièrement avantageux selon l'invention est caractérisé en ce que la solution aqueuse contenant le produit initialement hygroscopique, notamment le lactulose et le fluide cryogénique est pulvérisée à une pression d'environ 2 × 10⁶ Pa à environ 2 × 10⁷ Pa.

La présente invention concerne un procédé de préparation tel que défini ci-dessus, caractérisé en ce que l'étape de séchage par atomisation et de stabilisation primaire conduisant à un mélange pulvérisé est suivie d'une étape de stabilisation secondaire par refroidissement dudit mélange pulvérisé par de l'air secondaire, partiellement déshydraté, notamment introduit à contre courant par rapport à l'air chaud de séchage.

La présente invention consiste donc à associer un système de séchage de l'air par un système Roue Dessicative type Munters, permettant d'améliorer la productivité du procédé en disposant d'un air déshydraté à 1 g d'eau par kg d'air, sans que ce système soit obligatoire pour conduire le procédé, un système de séchage partiel de l'air par batterie de refroidissement permettant d'atteindre des taux d'humidité résiduelle suffisants de 4 à 5g d'eau par kg d'air.

Selon un mode de réalisation préféré, le procédé de l'invention est caractérisé en ce que la composition pulvérulente non hygroscopique de lactulose, obtenue à l'issue de l'étape de séchage par atomisation et de stabilisation primaire et de l'étape de stabilisation secondaire, effectuées dans une tour d'atomisation, est introduite dans un ou plusieurs cyclone(s).

Le procédé de la présente invention est également caractérisé en ce que la composition pulvérulente non hygroscopique de lactulose, est récupérée en bas de la tour d'atomisation, sous forme de poudre microgranulée dont la granulométrie moyenne peut varier d'environ 100 µm à environ 500 µm.

Le procédé de la présente invention est également caractérisé en ce que la composition pulvérulente non hygroscopique de lactulose, est récupérée à la sortie de l'un des cyclones.

La présente invention concerne également un procédé tel que défini ci-dessus, caractérisé en ce que la solution aqueuse contenant au moins un produit initialement hygroscopique, notamment le lactulose, et un fluide cryogénique est co-séchée par atomisation avec une substance en poudre hygroscopique.

Il existe une technologie dite de séchage par atomisation et/ou de co-séchage associant au séchage par atomisation de liquides, une ou plusieurs injections de formes sèches, qui permet de produire des poudres d'ingrédients purs ou de mélanges d'ingrédients directement compressibles, tels que des sucres, des compléments alimentaires minéraux et vitaminés. On peut par exemple envisager la pulvérisation d'une solution de lactulose associée à l'injection d'une poudre de lactulose pur ou en combinaison avec d'autres sucres à propriétés prébiotiques (fructo-oligosaccharides, fructose, lactose), des gommes arabiques, de l'inuline et des mélanges prébiotiques-probiotiques (poudres de lactulose, bactéries lactiques, levures). Cette technologie offre l'avantage d'obtenir des poudres homogènes à compression directe. La compression directe est d'autant plus utilisée qu'elle permet d'obtenir des formes solides (comprimés, galets, tablettes) sans utilisation de liants ou d'étape supplémentaire de granulation.

Selon un mode de réalisation avantageux, le procédé de l'invention est caractérisé en ce que la substance en poudre hygroscopique est choisie parmi des produits organiques dont la masse moléculaire moyenne est inférieure à environ 1 000 Da, notamment comprenant au moins 50% en poids de glucides, tels que le lactulose, les fructo-oligosaccharides, le fructose, le saccharose, le glucose ou des mélanges de ceux-ci, des polyols tels que le sorbitol, le maltitol ou le xylitol, des compositions à base de miel, des produits dérivés de l'extraction du lactose, le lactosérum ou ses dérivés, des mélanges de sucres et édulcorants, tels que des mélanges de fructo-oligosaccharides et d'aspartame, d'acésulfame ou de rhamnose.

Parmi les produits hygroscopiques, on peut également citer les extraits de plantes hygroscopiques tels que du sérum de luzerne obtenu après extraction des protéines, le contenu cytoplasmique de cellules de plantes fraîches, des extraits d'artichaut ou des extraits polyphénoliques végétaux (notamment raisin ou pomme), les mélanges associant un ou plusieurs probiotiques (et notamment ceux de la famille des Lactobacillus et des Bacillus), et un ou plusieurs actifs d'intérêt nutritionnel et notamment les prébiotiques (notamment le lactulose, les fructo-oligossacharides, le rhamnose), les acides gras poly-insaturés (tels que des extraits d'huile de poissons riches en ω3), les polyphénols (notamment les catéchols et les extraits de pépin de raisin), et les extraits de levure.

La présente invention concerne également un procédé de préparation en continu d'une composition pulvérulente non hygroscopique, ledit procédé étant caractérisé en ce qu'il comprend les étapes suivantes :
- une étape de chauffage d'une solution aqueuse initiale contenant un produit initialement hygroscopique, le lactulose présentant une température de transition vitreuse de 10°C à 110°C, à une température d'environ 50°C à environ 85°C, de préférence d'environ 65°C à environ 80°C, afin d'obtenir une solution aqueuse initiale chauffée,
- une étape de dissolution d'un fluide cryogénique, notamment d'un fluide cryogénique alimentaire ou d'un mélange de fluides cryogéniques, dans ladite solution aqueuse initiale chauffée, ledit fluide cryogénique étant notamment choisi parmi l'anhydride carbonique, l'azote ou l'air liquide, et étant à une pression comprise d'environ 10⁵ Pa à environ 20 × 10⁵ Pa, de préférence d'environ 4 × 10⁵ Pa à environ 12 × 10⁵ Pa, afin d'obtenir une solution aqueuse contenant ledit produit hygroscopique et un fluide cryogénique,
- une étape de pulvérisation simultanée d'un agent anti-agglomérant et de la solution aqueuse contenant ledit produit hygroscopique et un fluide cryogénique, afin d'obtenir un mélange pulvérisé,
- une étape de séchage par atomisation dudit mélange pulvérisé avec de l'air chaud à une température d'environ 100°C à environ 250°C, de préférence d'environ 115°C à environ 150°C, notamment dans une tour d'atomisation, afin d'obtenir une poudre hygroscopique, partiellement séchée et non stabilisée, et de stabilisation primaire, correspondant à un refroidissement et à un refroidissement instantané par la détente du fluide cryogénique de ladite poudre hygroscopique, partiellement séchée et non stabilisée, afin d'obtenir une composition dudit produit hygroscopique sous la forme d'une poudre stabilisée non hygroscopique,
- une étape de stabilisation secondaire de la poudre stabilisée non hygroscopique obtenue à l'étape précédente, correspondant à un refroidissement dans l'air partiellement séché.

La présente invention permet donc de produire des poudres de meilleure qualité bactériologique par l'effet bactériostatique constaté lié à la présence du fluide cryogénique.

La présente invention concerne également une composition pulvérulente non hygroscopique de lactulose telle qu'obtenue par le procédé tel que défini ci-dessus, éventuellement en mélange avec des produits ayant des propriétés nutritionnelles et/ou thérapeutiques.

Le caractère non collant de la composition pulvérulente de lactulose de l'invention peut être vérifié par les tests décrits dans l'article de Papadakis et Bahu, "The sticky issues of drying", dans Drying Technology, 10(4), 817-837 (1992).

Les compositions pulvérulentes de lactulose de l'invention peuvent être redissoutes dans l'eau.

La présente invention permet de fournir un procédé de séchage par atomisation d'une solution de lactulose qui permet de produire des compositions pulvérulentes à haute concentration en lactulose sans adjonction de support, à partir de solutions de lactulose technique concentrées issues des usines de fabrication, présentant des propriétés physiques nouvelles et surprenantes : ces compositions ne présentent plus le caractère d'hygroscopicité et d'instabilité immédiate des poudres de l'état de la technique, et présentent en comparaison une stabilité remarquable dans des conditions de stockage à l'air ambiant ; elles offrent en outre des propriétés de coulabilité et de mouillabilité excellentes. Il est un autre objet de l'invention qui tient dans le fait nouveau que les poudres obtenues sont directement compressibles, et permettent de produire des comprimés, tablettes et galets sans passer par une phase de granulation supplémentaire.

### LÉGENDE DES FIGURES

Le cercle A représente la solution aqueuse initiale contenant au moins un produit initialement hygroscopique ; le rectangle (2) représente un réchauffeur ; le cercle B représente l'introduction du fluide cryogénique ou d'un mélange de fluides cryogéniques et le rectangle (3) représente l'étape de dissolution dudit fluide cryogénique ou dudit mélange de fluides cryogéniques dans ladite solution aqueuse initiale, afin d'obtenir une solution aqueuse contenant au moins un produit initialement hygroscopique et un fluide cryogénique ou un mélange de fluides cryogéniques. Le rectangle (4) représente une pompe à haute pression utilisée pour pulvériser ladite solution aqueuse dans la tour d'atomisation (1) via une ou plusieurs buses (5). Le cercle C représente la pulvérisation de l'agent anti-agglomérant via un doseur de poudre (6)

Le cercle D représente l'introduction de l'air chaud (température de 100°C à 250°C) pour l'étape d'atomisation, via un ventilateur (7).

Le cercle E représente l'introduction de l'air secondaire, partiellement déshydraté (température de 100°C à 250°C), via un ventilateur (8).

Le rectangle (9) représente un cyclone ; le cercle F représente la récupération du produit final par le cyclone, c'est-à-dire la composition pulvérulente non hygroscopique et le cercle G représente l'évacuation de l'air de sortie du cyclone.

Le rectangle (10) représente un lit fluidisé vibré externe ; le cercle H représente la récupération du produit final à la sortie dudit lit fluidisé, c'est-à-dire la composition pulvérulente non hygroscopique.

Le cercle I représente l'addition en zone de pulvérisation d'une substance en poudre hygroscopique et le rectangle (11) correspond au dispositif d'injection constitué principalement d'un doseur de poudre.

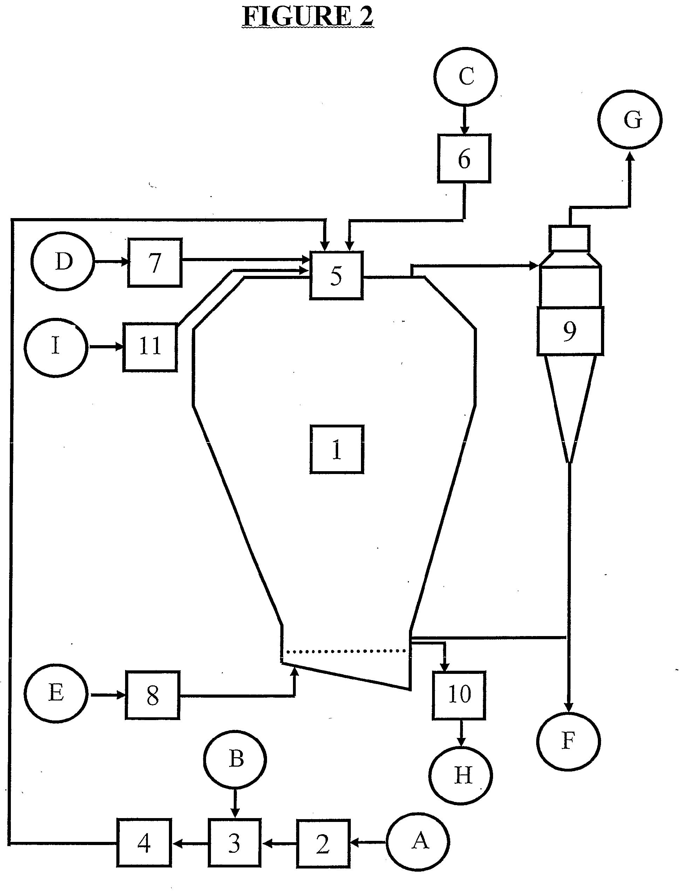

La Figure 2 est un schéma de principe du procédé de l'invention, mis en oeuvre dans une tour d'atomisation à multiples effets. Les cercles A, B, C, D, E, F, G, H et I, ainsi que les rectangles (1) à (11) ont la même signification indiquée que celle de la Figure 1.

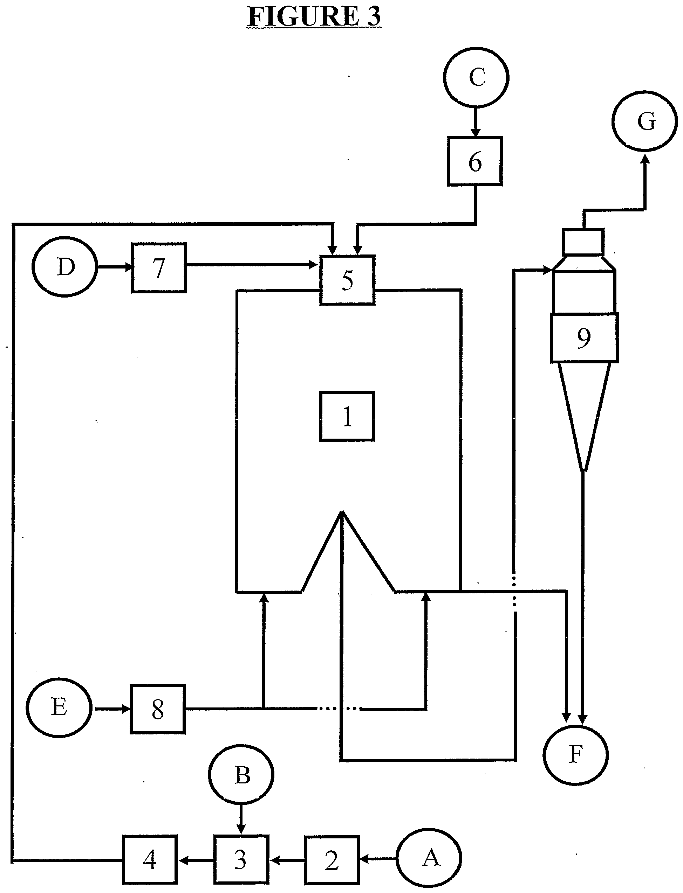

La Figure 3 est un schéma de principe du procédé de l'invention, mis en oeuvre dans une tour d'atomisation à fond W. Les cercles A, B, C, D, E, F et G, ainsi que les rectangles (1) à (9) ont la même signification indiquée que celle de la Figure 1.

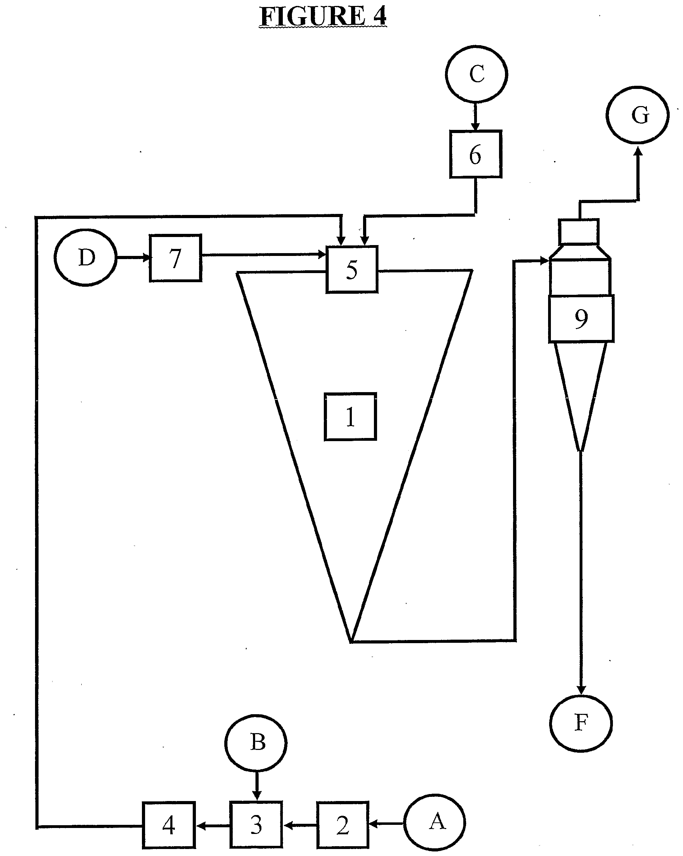

La Figure 4 est un schéma de principe du procédé de l'invention, mis en oeuvre dans une tour simple effet. Les cercles A, B, C, D, F et G, ainsi que les rectangles (1) à (7) et (9) ont la même signification indiquée que celle de la Figure 1.
La Figure 5A représente un champ d'un produit témoin A de poudre de lactulose n'utilisant pas le procédé de l'invention (avec un grossissement de 200 fois). On note une dispersion élevée des particules toutes sphériques.
La Figure 5B représente un champ d'un produit témoin de poudre de lactulose n'utilisant pas le procédé de l'invention (avec un grossissement de 500 fois). On note que les particules unitaires ont la même forme avec un état lisse de la surface avec des particules de 30 à 100 microns.
La Figure 6A représente un champ d'un produit de l'exemple 2 de l'invention (voir ci-après) de poudre de lactulose (avec un grossissement de 200 fois). On note une dispersion élevée des particules toutes sphériques mais de taille plus élevée que le témoin A. Les particules ont des nervures caractéristiques.
La Figure 6B représente un champ d'un produit de l'exemple 2 de l'invention de poudre de lactulose (avec un grossissement de 500 fois). On note que les particules unitaires sont sphériques mais de taille plus élevée que le témoin A. Les particules ont un état nervuré et gonflé caractéristique, avec une présence plus marquée de l'orifice caractéristique du dégazage du séchage par atomisation visualisée à la surface de la particule.
La Figure 7 représente une particule avec un grossissement de 500 fois avec un état de surface nervuré avec un élément caractéristique de l'orifice de dégazage en forme de "tâche solaire" avec des traits bruns en forme d'étoile.

### EXEMPLES

### EXEMPLE 1

### Séchage par atomisation à multiples effets dans une tour en configuration modifiée simple effet

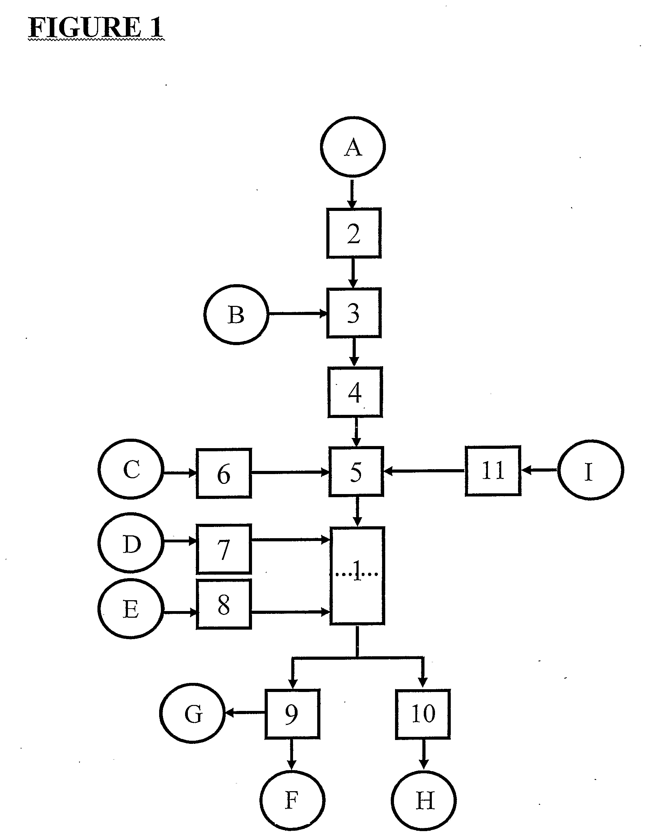

L'équipement utilisé dans cet exemple est une tour de séchage par atomisation à multiples effets (1) (voir figure 1) dont l'originalité du procédé est d'utiliser préférentiellement la tour en configuration modifiée simple effet. Les gouttes formées lors de la pulvérisation de la solution de lactulose sont séchées dans la chambre d'atomisation par de l'air chaud, la température de l'air entrant étant fixée à 116°C.

Dans cet exemple, le fluide cryogénique est de l'anhydride carbonique.

Une solution de lactulose dont la pureté en lactulose est de 70% exprimé en poids de lactulose sur poids de matières sèches, et dont la concentration en extrait sec de la solution est de 64% exprimé en poids de matières sèches sur le poids de la solution, est transférée à un débit de 140 kg/h par une pompe de gavage dans un réchauffeur à échangeur eau chaude (2) pour atteindre une température de 61°C et subit une carbonatation en continu (3), la pression du mélange carbonaté étant de 10 bars, avant d'être pulvérisée au moyen d'une pompe haute pression (4) à une pression d'environ 200 bars (2 × 10⁷ Pa) par une canne (5) à une buse mono fluide. Un dosage en continu de l'anti-agglomérant (6), de préférence une silice colloïdale, est effectué à un taux de 0,2% exprimé en poids de silice sur poids de l'extrait sec de la solution, par injection à proximité de la zone de pulvérisation.

La température du lit fluidisé est ajustée pour maintenir la poudre à une température de 20°C.

La température de l'air de sortie est choisie à 75°C ; cette alimentation en air dit secondaire (8) est désaturé partiellement par un système de batterie froide à contre courant d'eau glycolée, l'air obtenu présentant une humidité résiduelle de 5g d'eau par kg d'air.

La reprise d'air de la poudre finale est réalisée sous cyclone en choisissant un système de transport phase dense et la reprise de la poudre est effectuée par transport phase dense.

### Test Flodex™ de détermination de l'indice de fluidité d'une poudre

L'indice de fluidité Flodex™ est égal au diamètre de l'orifice du plus petit disque par lequel la poudre est tombée trois fois de manière consécutive (appareillage Hansen Research Corporation).

L'écoulement est alors déterminé selon l'échelle suivante en fonction de l'indice de fluidité trouvé.

| Indice de fluidité en mm | 4 - 7 | 8 - 12 | 14 - 18 | 20 - 26 | 28 - 34 |
|---|---|---|---|---|---|
| Ecoulement | Excellent | Bon | Moyen | Passable | Mauvais |

### Test de mouillabilité

La mouillabilité est l'aptitude d'une poudre à être mouillée. Elle correspond au temps nécessaire (en secondes) à une certaine quantité de poudre pour pénétrer dans l'eau à travers sa surface libre au repos.

### Mode opératoire

On verse 100 ml d'eau dans un bécher et on place un entonnoir (en matière antistatique) de façon à ce qu'il s'appuie sur le bord supérieur du bécher. La température de l'eau est maîtrisée (20°C ± 2°C).

On obture ensuite l'ouverture inférieure de l'entonnoir et on place autour de l'obturateur la quantité de poudre pesée (la quantité d'échantillon destinée à l'analyse doit correspondre à la concentration de la poudre dans l'eau à laquelle le produit donné va être utilisé). Enfin, on soulève l'obturateur et on mesure alors le temps écoulé jusqu'à ce que toute la poudre soit mouillée.

Les caractéristiques des poudres fabriquées selon l'exemple 1 sont données dans le tableau 1.

**Tableau 1**

| | |
|---|---|
| Humidité Produit fini | 2% |
| Densité apparente | 300 g/l |
| Ecoulement (flowdex) | 20 |
| Aw Activité d'eau | 0,206 |
| D(v,0.5) | 40 µm |
| Granulométrie moyenne laser | |

On rappelle ici que la densité apparente désigne la densité mesurée de la poudre ; il s'agit donc du rapport entre la masse de la poudre et le volume occupé par la poudre.

### EXEMPLE 2

### Séchage par atomisation à multiples effets dans une tour en configuration modifiée simple effet

Dans cet exemple, on utilise l'équipement de la figure 1, c'est-à-dire une tour de séchage par atomisation à multiples effets (1) dont l'originalité du procédé est d'utiliser préférentiellement la tour en configuration modifiée simple effet. Les gouttes formées lors de la pulvérisation de la solution de lactulose sont séchées dans la chambre d'atomisation par de l'air chaud, la température de l'air entrant étant fixée à 120°C.

Dans cet exemple, le fluide cryogénique est de l'anhydride carbonique.

Une solution de lactulose dont la pureté en lactulose est de 70% exprimé en poids de lactulose sur poids de matières sèches, et dont la concentration en extrait sec de la solution est de 65% exprimé en poids de matières sèches sur poids de la solution, est transférée à un débit de 230 kg/h par une pompe de gavage dans un réchauffeur à échangeur eau chaude (2) pour atteindre une température de 60°C et subit une carbonatation en continu (3), la pression du mélange carbonaté étant de 10 bars (10⁶ Pa), avant d'être pulvérisée au moyen d'une pompe haute pression (4) à une pression de 160 bars (1,6 × 10⁷ Pa) par une canne (5) à une buse mono fluide. Un dosage en continu de l'anti-agglomérant (6), de préférence une silice colloïdale à un taux de 1% exprimé en poids de silice sur poids de l'extrait sec de la solution, est effectué par injection à proximité de la zone de pulvérisation.

La température du lit fluidisé est ajustée pour maintenir la poudre à une température de 21°C.

La température de l'air de sortie est choisie à 76°C ; cette alimentation en air dit secondaire (8) est désaturé partiellement par un système de batterie froide à contre courant d'eau glycolée, l'air obtenu présentant une humidité résiduelle de 5g d'eau par kg d'air.

La reprise d'air de la poudre finale est réalisée sous cyclone en choisissant un système de transport phase dense et la reprise de la poudre est effectuée par transport phase dense.

Les caractéristiques des poudres fabriquées selon cet exemple sont données dans le tableau 2.

**Tableau 2**

| | |
|---|---|
| Humidité Produit fini | 1,5% |
| Densité apparente | 490 g/l |
| Ecoulement (flowdex) | 20 |
| Aw Activité d'eau | 0,173 |
| D(v,0.5) | 71 µm |
| Granulométrie moyenne laser | |

### EXEMPLE 3

### Séchage par atomisation à multiples effets dans une tour en configuration modifiée simple effet

Dans cet exemple, l'équipement utilisé est celui de la figure 1, c'est-à-dire une tour de séchage par atomisation à multiples effets (1) dont l'originalité du procédé est d'utiliser préférentiellement la tour en configuration multiple effet. Les gouttes formées lors de la pulvérisation de la solution de lactulose sont séchées dans la chambre d'atomisation par de l'air chaud, la température de l'air entrant étant fixée à 120°C.

Dans cet exemple, le fluide cryogénique est de l'anhydride carbonique.

Une solution de lactulose dont la pureté en lactulose est de 70% exprimé en poids de lactulose sur poids de matières sèches, et dont la concentration en extrait sec de la solution est de 70% exprimé en poids de matières sèches sur poids de la solution, est transférée à un débit de 171 kg/h par une pompe de gavage dans un réchauffeur à échangeur eau chaude (2) pour atteindre une température de 60°C et subit une carbonatation en continu (3), la pression du mélange carbonaté étant de 10 bars (10⁶ Pa), avant d'être pulvérisée au moyen d'une pompe haute pression (4) à une pression de 114 bars (1,14 × 10⁷ Pa) par une canne (5) à une buse mono fluide. Un dosage en continu de l'anti-agglomérant (6), de préférence une silice colloïdale à un taux de 0,5% exprimée en poids de silice sur poids de l'extrait sec de la solution, est effectué par injection à proximité de la zone de pulvérisation.

La température du lit fluidisé est ajustée pour maintenir la poudre à une température de 18°C.

La température de l'air de sortie est choisie à 76°C ; cette alimentation en air dit secondaire (8) est désaturé partiellement par un système de batterie froide à contre courant d'eau glycolée, l'air obtenu présentant une humidité résiduelle de 5g d'eau par kg d'air.

La stabilisation de la poudre finale se fait par passage sur un vibro-fluidiseur équipé de deux sections d'air chauffé désaturé à 5g d'eau par kg d'air, dont la température sur la première section est de 44°C et de 33°C sur la deuxième section. La poudre est extraite en sortie de vibro-fluidiseur et tamisée.

Les caractéristiques des poudres fabriquées selon cet exemple sont données dans le tableau 3.

**Tableau 3**

| | |
|---|---|
| Humidité Produit fini | 2,5% |
| Densité apparente | 420 g/l |
| Ecoulement (flowdex) | 5 |
| Aw Activité d'eau | 0,276 |
| D(v,0.5) | 300 µm |
| Granulométrie moyenne laser | |

La poudre micro-granulée présente en outre une granulométrie centrée sur 350 µm, avec 0 particule d'une taille supérieure à 800 µm et 5% de particules inférieures à 80 µm, ce qui confère à la poudre des caractéristiques excellentes de coulabilité facilitant les opérations de dosage avec une bonne précision, et sans présence de poussières limitant ainsi les risques de contaminations croisées.

### EXEMPLE 4

### Séchage par atomisation dans une tour à fond W

Dans cet exemple, l'équipement utilisé est une tour à fond W dont l'originalité du procédé est d'utiliser la tour à fond W ou tour deux temps préférentiellement en configuration de tour simple effet. Les gouttes formées lors de la pulvérisation de la solution de lactulose sont séchées dans la chambre d'atomisation par de l'air chaud, la température de l'air entrant étant fixée à 106°C.

Dans cet exemple, le fluide cryogénique est de l'anhydride carbonique.

Une solution de lactulose dont la pureté en lactulose est de 70% exprimé en poids de lactulose sur poids de matières sèches, et dont la concentration en extrait sec de la solution est de 66% exprimé en poids de matières sèches sur poids de la solution, est transférée par une pompe de gavage dans un réchauffeur à effet Joule (2) de type Actijoule® pour atteindre une température d'environ 70°C et subit une carbonatation en continu (3), la pression du mélange carbonaté étant de 10 bars (10⁶ Pa), avant d'être pulvérisée au moyen d'une pompe haute pression (4) à une pression de 40 bars (4 × 10⁶ Pa) par une canne (5) à buse mono fluide. Un dosage en continu de l'anti-agglomérant (6), de préférence une silice colloïdale à un taux de 0,5% exprimée en poids de silice sur poids de l'extrait de la solution, est effectué par injection à proximité de la zone de pulvérisation.

La température du lit fluidisé est ajustée pour maintenir la poudre à une température de 25°C.

La température de l'air de sortie est choisie à 75°C ; cette alimentation en air dit secondaire (8) est désaturé partiellement par un système de batterie froide à contre courant d'eau glycolée, l'air obtenu présentant une humidité résiduelle de 5g d'eau par kg d'air.

La reprise de la poudre finale est réalisée sous cyclone utilisant un système de transport sous vide.

Les caractéristiques des poudres fabriquées selon cet exemple 4 sont données dans le tableau 4.

**Tableau 4**

| | |
|---|---|
| Humidité Produit fini | 2,7% |
| Densité apparente | 656 g/l |
| Ecoulement (flowdex) | 7 |
| D(v,0.5) | 60 µm |
| Granulométrie moyenne | |

Les valeurs indiquées pour l'indice de fluidité (flowdex) indiquent une poudre présentant des propriétés excellentes à bonnes d'écoulement (voir exemple 1).

### EXEMPLE 5

### Séchage par atomisation dans une tour à fond W

Dans cet exemple, l'équipement utilisé est une tour à fond W dont l'originalité du procédé est d'utiliser la tour à fond W ou tour deux temps préférentiellement en configuration de tour simple effet. Les gouttes formées lors de la pulvérisation de la solution de lactulose sont séchées dans la chambre d'atomisation par l'air chaud, la température de l'air entrant étant fixée à 106°C.

Dans cet exemple, le fluide cryogénique est de l'anhydride carbonique.

Une solution de lactulose dont la pureté en lactulose est de 70% exprimé en poids de lactulose sur poids de matières sèches, et dont la concentration en extrait sec de la solution est de 66% exprimé en poids de matières sèches sur poids de la solution, est transférée par une pompe de gavage dans un réchauffeur à effet Joule (2) de type Actijoule® pour atteindre une température d'environ 70°C et subit une carbonatation en continu (3), la pression du mélange carbonaté étant de 10 bars (10⁶ Pa), avant d'être pulvérisée au moyen d'une pompe haute pression (4) à une pression de 40 bars (4 × 10⁶ Pa) par une canne (5) à buse mono fluide. Un dosage en continu de l'anti-agglomérant (6), de préférence une silice colloïdale à un taux de 0,5% exprimée en poids de silice sur poids de l'extrait de la solution, est effectué par injection à proximité de la zone de pulvérisation.

La température du lit fluidisé est ajustée pour maintenir la poudre à une température de 25°C.

La température de l'air de sortie est choisie à 75°C ; cette alimentation en air dit secondaire (8) est désaturé partiellement par un système de batterie froide à contre courant d'eau glycolée, l'air obtenu présentant une humidité résiduelle de 5g d'eau par kg d'air.

La reprise de la poudre finale est réalisée sous cyclone utilisant un système de transport sous vide.

Les caractéristiques des poudres fabriquées selon cet exemple sont données dans le tableau 5.

**Tableau 5**

| | |
|---|---|
| Humidité Produit fini | 3% |
| Densité apparente | 484 g/l |
| Ecoulement (flowdeX) | 9 |
| D(v,0.5) | 40 µm |
| Granulométrie moyenne | |

### EXEMPLE 6

### Séchage par atomisation à multiples effets dans une tour en configuration multiple effet

Dans cet exemple, l'équipement utilisé est celui de la figure 1, c'est-à-dire une tour de séchage par atomisation à multiples effets (1) dont l'originalité du procédé est d'utiliser préférentiellement la tour en configuration multiple effet. La tour est par ailleurs équipée d'un système de co-séchage en tête de tour permettant d'assurer dans le même temps la pulvérisation en continu d'un ou plusieurs composants présentant des critères d'hygroscopicité reconnus. Le mélange formé par le brouillard de gouttes formées lors de la pulvérisation de la solution de lactulose et le nuage de particules solides soufflé au niveau de la buse de pulvérisation permet d'assurer une opération de co-séchage dans la chambre d'atomisation par de l'air chaud, la température de l'air entrant étant fixée à 120°C.

Dans cet exemple, le fluide cryogénique est de l'anhydride carbonique.

Une solution de lactulose dont la pureté en lactulose est de 70% exprimé en poids de lactulose sur poids de matière sèche, et dont la concentration en extrait sec de la solution est de 70% exprimé en poids de matières sèches sur poids de la solution, est transférée à un débit de 171 kg/h par une pompe de gavage dans un réchauffeur à échangeur eau chaude (2) pour atteindre une température de 60°C et subit une carbonatation en continu (3), la pression du mélange carbonaté étant de 10 bars (10⁶ Pa), avant d'être pulvérisée au moyen d'une pompe haute pression (4) à une pression de 114 bars (1,14 × 10⁷ Pa) par une canne (5) à une buse mono fluide. Un dosage en continu de l'anti-agglomérant (6), de préférence une silice colloïdale à un taux de 0,5% exprimé en poids de silice sur poids de l'extrait sec de la solution, est effectué par injection à proximité de la zone de pulvérisation. Un dosage en continu d'une poudre de lactose à un taux de 30% exprimé en poids de lactose sur poids de l'extrait sec de la solution, est effectué dans le même temps, par injection à proximité de la zone de pulvérisation.

La température du lit fluidisé est ajustée pour maintenir la poudre à une température de 18°C.

La température de l'air de sortie est choisie à 76°C ; cette alimentation en air dit secondaire (8) est désaturé partiellement par un système de batterie froide à contre courant d'eau glycolée, l'air obtenu présentant une humidité résiduelle de 5g d'eau par kg d'air.

La stabilisation de la poudre finale se fait par passage sur un vibro-fluidiseur équipé de deux sections d'air chauffé désaturé à 5g d'eau par kg d'air, dont la température sur la première section est de 44°C et de 33°C sur la deuxième section. La poudre est extraite en sortie de vibro-fluidiseur et tamisée.

Les caractéristiques des poudres fabriquées selon cet exemple sont données dans le tableau 6.

**Tableau 6**

| | |
|---|---|
| Humidité Produit fini | 2% |
| Densité apparente | 550 g/l |
| Ecoulement (flowdex) | 5 |
| Aw Activité d'eau | 0,28 |
| D(v,0.5) | 350 µm |
| Granulométrie moyenne laser | |

La poudre micro-granulée présente en outre une granulométrie centrée sur 375 µm, avec 0 particule d'une taille supérieure à 800 µm et 5% de particules inférieures à 80 µm, ce qui confère à la poudre des caractéristiques excellentes de coulabilité facilitant les opérations de dosage avec une bonne précision, et sans présence de poussières limitant ainsi les risques de contaminations croisées. En outre, la poudre présente une homogénéité parfaite, chaque particule étant uniformément constituée du mélange lactulose-lactose 70/30 dans les mêmes proportions que celles initialement définies lors des opérations de pulvérisation en tête de tour.

### EXEMPLE 7

### Séchage par atomisation à multiples effets dans une tour en configuration multiple effet, associé à un co-séchage

Dans cet exemple, l'équipement utilisé est celui de la figure 1, c'est-à-dire une tour de séchage par atomisation à multiples effets (1) dont l'originalité du procédé est d'utiliser préférentiellement la tour en configuration multiple effet. La tour est par ailleurs équipée d'un système de co-séchage en tête de tour permettant d'assurer dans le même temps la pulvérisation en continu d'un ou plusieurs composants présentant des critères d'hygroscopicité reconnus. Le mélange formé par le brouillard de gouttes formées lors de la pulvérisation de la solution de lactulose et le nuage de particules solides soufflé au niveau de la buse de pulvérisation permet d'assurer une opération de co-séchage dans la chambre d'atomisation par de l'air chaud, la température de l'air entrant étant fixée à 120°C.

Dans cet exemple, le fluide cryogénique est de l'anhydride carbonique.

Une solution de lactulose dont la pureté en lactulose est de 70% exprimé en poids de lactulose sur poids de matières sèches, et dont la concentration en extrait sec de la solution est de 70% exprimé en poids de matières sèches sur poids de la solution, est transférée à un débit de 171 kg/h par une pompe de gavage dans un réchauffeur à échangeur eau chaude (2) pour atteindre une température de 60°C et subit une carbonatation en continu (3), la pression du mélange carbonaté étant de 10 bars (10⁶ Pa), avant d'être pulvérisée au moyen d'une pompe haute pression (4) à une pression de 114 bars (1,14 × 10⁷ Pa) par une canne (5) à une buse mono fluide. Un dosage en continu de l'anti-agglomérant (6), de préférence une silice colloïdale à un taux de 0,5% exprimée en poids de silice sur poids de l'extrait sec de la solution, est effectué par injection à proximité de la zone de pulvérisation. Un dosage en continu d'une poudre de perméat de lactosérum doux à un taux de 30% exprimé en poids de lactose sur poids de l'extrait sec de la solution, est effectué dans le même temps, par injection à proximité de la zone de pulvérisation.

La température du lit fluidisé est ajustée pour maintenir la poudre à une température de 18°C.

La température de l'air de sortie est choisie à 76°C ; cette alimentation en air dit secondaire (8) est désaturé partiellement par un système de batterie froide à contre courant d'eau glycolée, l'air obtenu présentant une humidité résiduelle de 5g d'eau par kg d'air.

La stabilisation de la poudre finale se fait par passage sur un vibro-fluidiseur équipé de deux sections d'air chauffé désaturé à 5g d'eau par kg d'air, dont la température sur la première section est de 44°C et de 33°C sur la deuxième section. La poudre est extraite en sortie de vibro-fluidiseur et tamisée.

Les caractéristiques des poudres fabriquées selon cet exemple sont données dans le tableau 7.

**Tableau 7**

| | |
|---|---|
| Humidité Produit fini | 2% |
| Densité apparente | 550 g/l |
| Ecoulement (flowdex) | 6 |
| Aw Activité d'eau | 0,18 |
| D(v,0.5) | 350 µm |
| Granulométrie moyenne laser | |

La poudre micro-granulée présente en outre une granulométrie centrée sur 375 µm, avec 0 particule d'une taille supérieure à 800 µm et 5% de particules inférieures à 80 µm, ce qui confère à la poudre des caractéristiques excellentes de coulabilité facilitant les opérations de dosage avec une bonne précision, et sans présence de poussières limitant ainsi les risques de contaminations croisées. En outre, la poudre présente une homogénéité parfaite, chaque particule étant uniformément constituée du mélange lactulose-perméat de lactosérum dans un ratio lactulose/lactose de 70/30 dans les mêmes proportions que celles initialement définies lors des opérations de pulvérisation en tête de tour.

### EXEMPLE 8

### Mesure du caractère hygroscopiques des compositions pulvérulentes de l'invention

Le caractère hygroscopique et d'instabilité des poudres hygroscopiques et en particulier de lactulose est constaté facilement suivant un test où l'on place un échantillon de poudre fabriquée (témoins A et B) et des poudre issues de l'invention (exemples 1 à 5) dans une coupelle placée à température et humidité ambiantes (20°C et 45% d'humidité relative).

Les produits de l'art antérieur montrent une hygroscopicité marquée avec une agrégation dans un temps extrêmement court de 5 minutes d'exposition aboutissant à un mottage dans l'heure, au contraire des produits de l'invention, qui suivant les conditions d'expérience, conduisent soit à une stabilité avec une agrégation lente de 2 à 6 heures (poudres des exemples 1, 2 et 5) ou totalement stable (poudres des exemples 3 et 4).

De la même façon, le temps de mottage est nettement amélioré. Suivant les conditions d'expérience, les poudres présentent un mottage retardé de 4 à 8 heures (poudres des exemples 1, 2 et 5) ou sont totalement stables (poudres des exemples 3 et 4) après 72 heures d'exposition.

**TEST D'HYGROSCOSPICITE ET DE MOTTAGE**

| | Témoin A | Témoin B | Ex. 1 | Ex. 2 | Ex. 3 | Ex. 4 | Ex. 5 |
|---|---|---|---|---|---|---|---|
| Temps d'agrégation (en minutes) | 2 | 3 | 120 | 120 | stable pas d'agrégation après 72h | stable pas d'agrégation après 72h | 360 |
| Temps de mottage (en minutes) | 60 | 60 | 240 | 240 | stable pas de mottage après 72h | stable pas de mottage après 72h | 480 |
| % reprise en eau avant mottage | 0,7% | 0,6% | 3,2% | 2,1% | sans objet | sans objet | 1,2% |
| % reprise en eau après 8h | 1,6% | 2,5% | 4,6% | 3,4% | 2,6% | 3% | 1,2% |
| % reprise en eau après 72h | 1,9% | 3,7% | 4,1% | 4,1% | 3,2% | 3,3% | 0,9% |

Le temps d'agrégation est le temps pour lequel 1 à 2 grammes de poudre placés et répartis dans une coupelle aluminium (cellule à fond plat de 60 mm de diamètre et 20 mm de hauteur) dans une ambiance à 20°C et 45% d'humidité relative dans l'air, les particules adhèrent les unes aux autres.

Dans les mêmes conditions opératoires que pour le temps d'agrégation, le temps de mottage est le temps nécessaire pour que les particules agrégées entre elles soient refondues et forment un solide.

On note les pourcentages de reprise en eau en masse par rapport à la masse de l'échantillon de départ.

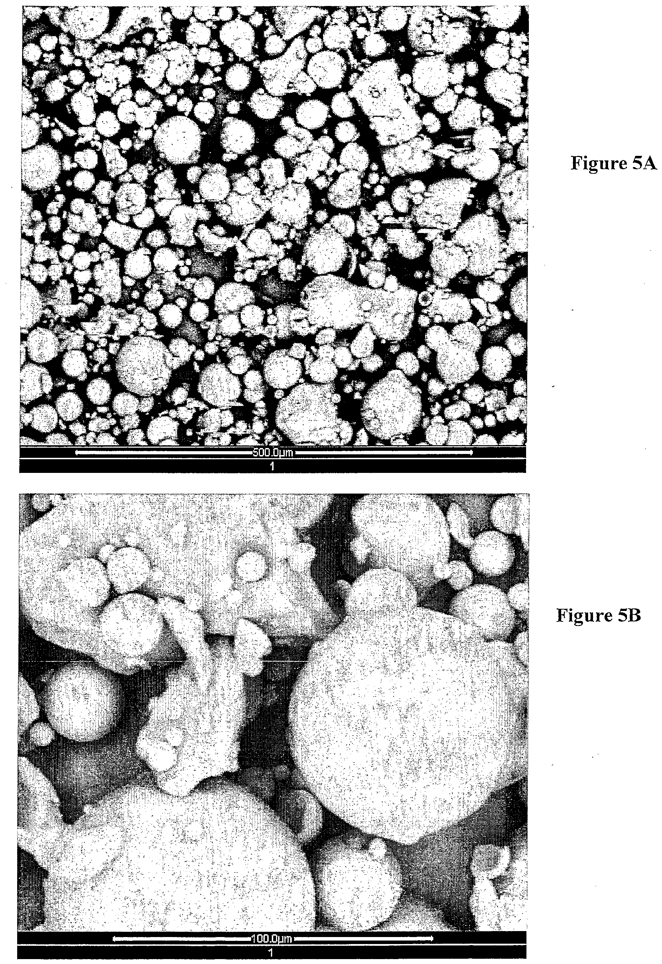

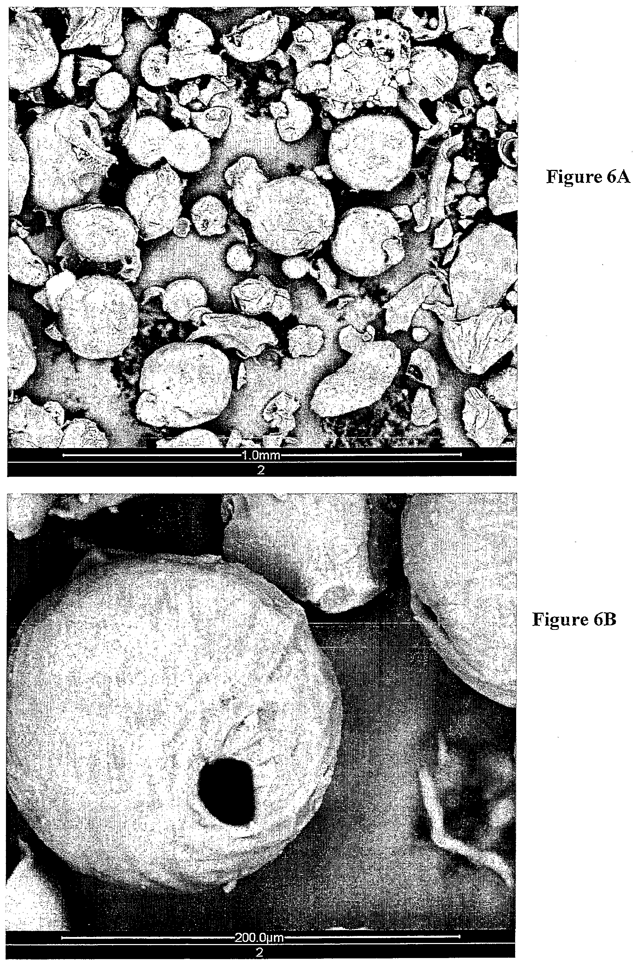

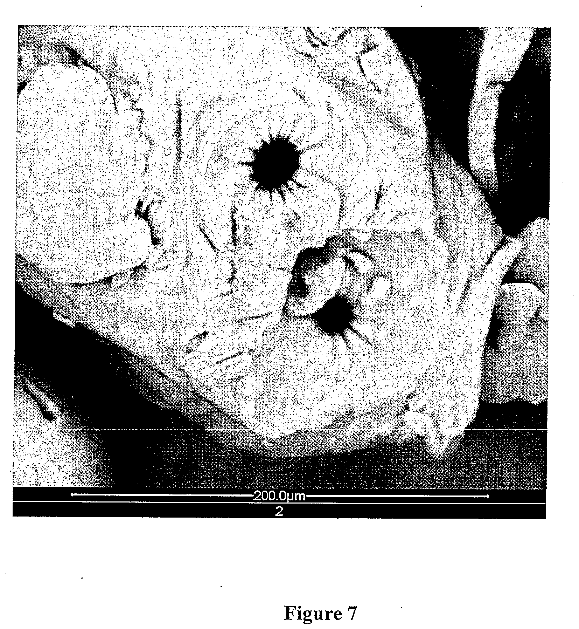

De la même façon, des clichés microphotographiques (voir Figures 5A, 5B, 6A, 6B et 7), effectués sur un microscope électronique à balayage, montrent clairement la différence de qualité des produits obtenus et la spécificité des poudres issues de l'invention, à savoir des particules nervurées et étirées lors du transfert des gaz avec une marque particulière au niveau du point de fuite d'évacuation de ces gaz caractéristique des poudres atomisées de la particule de « type tâche solaire ». :

## Revendications

1. Procédé de préparation d'une composition pulvérulente non hygroscopique comprenant une étape de séchage par atomisation, sans support d'atomisation, d'une solution aqueuse contenant au moins un produit initialement hygroscopique, et un fluide cryogénique, notamment un fluide cryogénique alimentaire, ou un mélange de fluides cryogéniques, notamment choisi parmi l'anhydride carbonique, l'azote, l'air liquide, ladite solution aqueuse étant obtenue par dissolution dudit fluide cryogénique dans une solution aqueuse initiale contenant ledit produit initialement hygroscopique, **caractérisé en ce que** le produit initialement hygroscopique est le lactulose.

2. Procédé de préparation selon la revendication 1, **caractérisé en ce que** l'étape de séchage par atomisation s'accompagne d'une stabilisation primaire résultant du refroidissement de la composition pulvérulente obtenue pendant l'étape de séchage par atomisation.

3. Procédé de préparation selon la revendication 2, **caractérisé en ce que** le refroidissement de la composition pulvérulente obtenue pendant l'étape de séchage par atomisation, a lieu dans une gamme de température inférieure à la température de transition vitreuse du produit hygroscopique, et **en ce que** la teneur en eau de ladite composition pulvérulente obtenue à l'issue de la susdite étape est inférieure à 7%, et est notamment de 1% à 4%.

4. Procédé de préparation selon l'une quelconque des revendications 1 à 3, **caractérisé en ce que** l'étape de séchage par atomisation est précédée d'une étape de pulvérisation simultanée d'un agent anti-agglomérant et de la solution aqueuse contenant le lactulose, et le fluide cryogénique alimentaire.

5. Procédé de préparation selon la revendication 4, **caractérisé en ce que** la concentration d'agent anti-agglomérant est inférieure à 0,5%, et varie de préférence de 0,1% à 0,3% en poids d'extrait sec de l'anti-agglomérant par rapport au poids d'extrait sec de la composition pulvérulente non hygroscopique de lactulose.

6. Procédé de préparation selon la revendication 4 ou 5, **caractérisé en ce que** l'agent anti-agglomérant est choisi parmi : la silice colloïdale, les silicates, le carbonate de magnésium, le calcium, le talc et le phosphate.

7. Procédé de préparation selon l'une quelconque des revendications 1 à 6, **caractérisé en ce que** l'étape de séchage par atomisation est effectuée avec de l'air chaud à une température de 100°C à 250°C, de préférence de 115°C à 150°C.

8. Procédé de préparation selon l'une quelconque des revendications 1 à 7, **caractérisé en ce que** la solution aqueuse initiale contenant le lactulose, est à une température de 50°C à 85°C, de préférence de 65°C à 80°C.

9. Procédé de préparation selon l'une quelconque des revendications 1 à 8, **caractérisé en ce que** la solution aqueuse initiale contenant le produit initialement hygroscopique, le lactulose, présente une concentration en matières sèches de 20% à 80% en poids de matière sèches par rapport au poids de la solution aqueuse initiale, et de préférence de 60% à 70%.

10. Procédé de préparation selon l'une quelconque des revendications 1 à 9, **caractérisé en ce que** la solution aqueuse initiale contenant le lactulose, contient de 20% à 100%, notamment de 50% à 100%, et de préférence de 60% à 80%, en poids de produit hygroscopique par rapport à la totalité du poids de matières sèches.

11. Procédé de préparation selon l'une quelconque des revendications 1 à 10, **caractérisé en ce que** le fluide cryogénique est à une pression comprise de 10⁵ Pa à 20 × 10⁵ Pa, de préférence de 4 × 10⁵ Pa à 12 × 10⁵ Pa.

12. Procédé de préparation selon l'une quelconque des revendications 1 à 11, **caractérisé en ce que** la solution aqueuse contenant le lactulose et le fluide cryogénique est pulvérisée à une pression de 2 × 10⁶ Pa à 2 × 10⁷ Pa.

13. Procédé de préparation selon l'une quelconque des revendications 1 à 12, **caractérisé en ce que** l'étape de séchage par atomisation et de stabilisation primaire conduisant à un mélange pulvérisé est suivie d'une étape de stabilisation secondaire par refroidissement dudit mélange pulvérisé par de l'air secondaire, partiellement déshydraté.

14. Procédé selon l'une quelconque des revendications 1 à 13, **caractérisé en ce que** la composition pulvérulente non hygroscopique de lactulose, obtenue à l'issue de l'étape de séchage par atomisation et de stabilisation primaire et de l'étape de stabilisation secondaire, effectuées dans une tour d'atomisation, est introduite dans un ou plusieurs cyclone(s).

15. Procédé selon l'une quelconque des revendications 1 à 14, **caractérisé en ce que** la composition pulvérulente non hygroscopique de lactulose, est récupérée en bas de la tour d'atomisation, sous forme de poudre microgranulée dont la granulométrie moyenne peut varier de 100 µm à 500 µm.

16. Procédé selon la revendication 1 à 14, **caractérisé en ce que** la composition pulvérulente non hygroscopique de lactulose, est récupérée à la sortie de l'un des cyclones.

17. Procédé selon l'une quelconque des revendications 1 à 16, **caractérisé en ce que** la solution aqueuse contenant le lactulose, et un fluide cryogénique est co-séchée par atomisation avec une substance en poudre hygroscopique.

18. Procédé selon la revendication 17, **caractérisé en ce que** la substance en poudre hygroscopique est choisie parmi des produits organiques dont la masse moléculaire moyenne est inférieure à 1 000 Da, notamment comprenant au moins 50% en poids de glucides, tels que le lactulose, les fructo-oligosaccharides, le fructose, le saccharose, le glucose ou des mélanges de ceux-ci, des polyols tels que le sorbitol, le maltitol ou le xylitol, des compositions à base de miel, des produits dérivés de l'extraction du lactose, le lactosérum ou ses dérivés, des mélanges de sucres et édulcorants, tels que des mélanges de fructo-oligosaccharides et d'aspartame, d'acésulfame ou de rhamnose.

19. Procédé de préparation en continu d'une composition pulvérulente non hygroscopique selon l'une quelconque des revendications 1 à 18, ledit procédé étant **caractérisé en ce qu'**il comprend les étapes suivantes :
- une étape de chauffage d'une solution aqueuse initiale contenant un produit initialement hygroscopique, le lactulose présentant une température de transition vitreuse de 10°C à 110°C, à une température de 50°C à 85°C, de préférence de 65°C à 80°C, afin d'obtenir une solution aqueuse initiale chauffée,
- une étape de dissolution d'un fluide cryogénique, notamment d'un fluide cryogénique alimentaire ou d'un mélange de fluides cryogéniques, dans ladite solution aqueuse initiale chauffée, ledit fluide cryogénique étant notamment choisi parmi l'anhydride carbonique, l'azote ou l'air liquide, et étant à une pression comprise de 10⁵ Pa à 20 × 10⁵ Pa, de préférence de 4 × 10⁵ Pa à 12 × 10⁵ Pa, afin d'obtenir une solution aqueuse contenant ledit produit hygroscopique et un fluide cryogénique,
- une étape de pulvérisation simultanée d'un agent anti-agglomérant et de la solution aqueuse contenant ledit produit hygroscopique et un fluide cryogénique, afin d'obtenir un mélange pulvérisé,
- une étape de séchage par atomisation dudit mélange pulvérisé avec de l'air chaud à une température de 100°C à 250°C, de préférence de 115°C à 150°C, notamment dans une tour d'atomisation, afin d'obtenir une poudre hygroscopique, partiellement séchée et non stabilisée, et de stabilisation primaire, de ladite poudre hygroscopique, partiellement séchée et non stabilisée, afin d'obtenir une composition dudit produit hygroscopique sous la forme d'une poudre stabilisée non hygroscopique,
- une étape de stabilisation secondaire de la poudre stabilisée non hygroscopique obtenue à l'étape précédente.

20. Procédé de préparation selon l'une des revendications 1 à 19, **caractérisé en ce que** le produit initialement hygroscopique est le lactulose, éventuellement en mélange avec des produits ayant des propriétés nutritionnelles et/ou thérapeutiques.

## Patentansprüche

1. Verfahren zur Herstellung einer nicht-hygroskopischen pulverförmigen Zusammensetzung, umfassend einen Schritt des Trocknens durch Zerstäuben, ohne Zerstäubungsträger, einer wässrigen Lösung, enthaltend mindestens ein anfänglich hygroskopisches Produkt und ein kryogenes Fluid, insbesondere ein kryogenes Nahrungsmittelfluid, oder eine Mischung kryogener Fluids, insbesondere ausgewählt aus Kohlensäure, Stickstoff, flüssiger Luft, wobei die wässrige Lösung durch Lösen des kryogenen Fluids in einer wässrigen Ausgangslösung, enthaltend das anfänglich hygroskopische Produkt, erhalten wird, **dadurch gekennzeichnet, dass** das anfänglich hygroskopische Produkt Lactulose ist.

2. Herstellungsverfahren nach Anspruch 1, **dadurch gekennzeichnet, dass** der Schritt des Trocknens durch Zerstäuben von einer primären Stabilisation begleitet wird, die aus dem Kühlen der pulverförmigen Zusammensetzung resultiert, welche während des Schritts des Trocknens durch Zerstäuben erhalten wird.

3. Herstellungsverfahren nach Anspruch 2, **dadurch gekennzeichnet, dass** das Kühlen der pulverförmigen Zusammensetzung, welche während des Schritts des Trocknens durch Zerstäuben erhalten wird, in einem Temperaturbereich unter der Glasübergangstemperatur des hygroskopischen Produkts stattfindet, und dadurch, dass der Wassergehalt der pulverförmigen Zusammensetzung, welche am Ende des genannten Schritts erhalten wird, kleiner ist als 7 % und insbesondere 1 % bis 4 % beträgt.

4. Herstellungsverfahren nach einem der Ansprüche 1 bis 3, **dadurch gekennzeichnet, dass** dem Schritt des Trocknens durch Zerstäuben ein Schritt des gleichzeitigen Pulverisierens eines Antiagglomerationsmittels und der wässrigen Lösung, die Lactulose und das kryogene Nahrungsmittelfluid enthält, vorausgeht.

5. Herstellungsverfahren nach Anspruch 4, **dadurch gekennzeichnet, dass** die Konzentration des Antiagglomerationsmittels kleiner ist als 0,5 % und vorzugsweise von 0,1 % bis 0,3 %, bezogen auf das Gewicht des Trockenextrakts des Antiagglomerationsmittels im Verhältnis zum Gewicht des Trockenextrakts der nicht-hygroskopischen pulverförmigen Zusammensetzung von Lactulose, variiert.

6. Herstellungsverfahren nach Anspruch 4 oder 5, **dadurch gekennzeichnet, dass** das Antiagglomerationsmittel ausgewählt wird aus: kolloidalem Siliciumdioxid, Silikaten, Magnesiumcarbonat, Calcium, Talk und Phosphat.

7. Herstellungsverfahren nach einem der Ansprüche 1 bis 6, **dadurch gekennzeichnet, dass** der Schritt des Trocknens durch Zerstäuben mit heißer Luft bei einer Temperatur von 100°C bis 250°C, vorzugsweise von 115°C bis 150°C, durchgeführt wird.

8. Herstellungsverfahren nach einem der Ansprüche 1 bis 7, **dadurch gekennzeichnet, dass** die wässrige Ausgangslösung, die Lactulose enthält, auf einer Temperatur von 50°C bis 85°C, vorzugsweise von 65°C bis 80°C, ist.

9. Herstellungsverfahren nach einem der Ansprüche 1 bis 8, **dadurch gekennzeichnet, dass** die wässrige Ausgangslösung, die das anfänglich hygroskopische Produkt enthält, Lactulose, eine Konzentration an trockenen Stoffen von 20 % bis 80 %, bezogen auf das Gewicht der trockenen Stoffe im Verhältnis zum Gewicht der wässrigen Ausgangslösung, und vorzugsweise von 60 % bis 70 %, aufweist.

10. Herstellungsverfahren nach einem der Ansprüche 1 bis 9, **dadurch gekennzeichnet, dass** die wässrige Ausgangslösung, die Lactulose enthält, von 20 % bis 100 %, insbesondere von 50 % bis 100 %, und vorzugsweise von 60 % bis 80 %, bezogen auf das Gewicht des hygroskopischen Produkts im Verhältnis zur Gesamtheit des Gewichts der trockenen Stoffe, enthält.

11. Herstellungsverfahren nach einem der Ansprüche 1 bis 10, **dadurch gekennzeichnet, dass** das krygoene Fluid auf einem Druck von 10⁵ Pa bis 20 x 10⁵ Pa, vorzugsweise von 4 x 10⁵ Pa bis 12 x 10⁵ Pa, ist.

12. Herstellungsverfahren nach einem der Ansprüche 1 bis 11, **dadurch gekennzeichnet, dass** die wässrige Lösung, die Lactulose und das kryogene Fluid enthält, bei einem Druck von 2 x 10⁶ Pa bis 2 x 10⁷ Pa pulverisiert wird.

13. Herstellungsverfahren nach einem der Ansprüche 1 bis 12, **dadurch gekennzeichnet, dass** der Schritt des Trocknens durch Zerstäuben und der primären Stabilisierung, der zu einer pulverisierten Mischung führt, von einem Schritt einer sekundären Stabilisierung durch Kühlen der pulverisierten Mischung mit sekundärer Luft, die teilweise dehydratisiert ist, gefolgt wird.

14. Verfahren nach einem der Ansprüche 1 bis 13, **dadurch gekennzeichnet, dass** die nicht-hygroskopische pulverförmige Zusammensetzung von Lactulose, welche aus dem Schritt des Trocknens durch Zerstäuben und der primären Stabilisierung und aus dem Schritt der sekundären Stabilisierung erhalten wird, die in einem Zerstäubungsturm durchgeführt werden, in einen oder mehrere Zyklone eingebracht wird.

15. Verfahren nach einem der Ansprüche 1 bis 14, **dadurch gekennzeichnet, dass** die nicht-hygroskopische pulverförmige Zusammensetzung von Lactulose am Boden des Zerstäubungsturms in der Form eines mikrogranulierten Pulvers gewonnen wird, dessen mittlere Granulometrie von 100 µm bis 500 µm variieren kann.

16. Verfahren nach einem der Ansprüche 1 bis 14, **dadurch gekennzeichnet, dass** die nicht-hygroskopische pulverförmige Zusammensetzung von Lactulose am Ausgang eines der Zyklone gewonnen wird.

17. Verfahren nach einem der Ansprüche 1 bis 16, **dadurch gekennzeichnet, dass** die wässrige Lösung, die Lactulose und ein krygones Fluid enthält, durch Zerstäuben mit einer Substanz aus einem hygroskopischen Pulver co-getrocknet wird.

18. Verfahren nach Anspruch 17, **dadurch gekennzeichnet, dass** die Substanz aus einem hygroskopischen Pulver ausgewählt wird aus organischen Produkten, deren mittlere Molekularmasse kleiner ist als 1 000 Da, insbesondere umfassend mindestens 50 Gew.-% Glucide, wie Lactulose, Fructooligosaccharide, Fructose, Saccharose, Glucose oder Mischungen davon, Polyole, wie Sorbit, Maltit oder Xylit, Zusammensetzungen auf der Basis von Honig, Produkten, die aus der Extraktion von Lactose stammen, Lactoserum und seinen Derivaten, Mischungen von Zuckern und Süßstoffen, wie Mischungen von Fructooligosacchariden und Aspartam, Acesulfam oder Rhamnose.

19. Verfahren zur kontinuierlichen Herstellung einer nicht-hygroskopischen pulverförmigen Zusammensetzung nach einem der Ansprüche 1 bis 18, wobei das Verfahren **dadurch gekennzeichnet ist, dass** es die folgenden Schritte umfasst:
- einen Schritt des Erhitzens einer wässrigen Ausgangslösung, die ein anfänglich hygroskopisches Produkt, Lactulose, mit einer Glasübergangstemperatur von 10°C bis 110°C, enthält, bei einer Temperatur von 50°C bis 85°C, vorzugsweise von 65°C bis 80°C, um eine erhitzte wässrige Ausgangslösung zu erhalten,
- einen Schritt des Lösens eines kryogenen Fluids, insbesondere eines kryogenen Nahrungsmittelfluids oder einer Mischung kryogener Fluids, in der erhitzten wässrigen Ausgangslösung, wobei das kryogene Fluid insbesondere ausgewählt wird aus Kohlensäure, Stickstoff oder flüssiger Luft, und auf einem Druck von 10⁵ Pa bis 20 x 10⁵ Pa, vorzugsweise von 4 x 10⁵ Pa bis 12 x 10⁵ Pa, ist, um eine wässrige Lösung zu erhalten, die das hygroskopische Produkt und ein kryogenes Fluid enthält,
- einen Schritt des gleichzeitigen Pulverisierens eines Antiagglomerationsmittels und der wässrigen Lösung, die das hygroskopische Produkt und ein kryogenes Fluid enthält, um eine pulverisierte Mischung zu erhalten,
- einen Schritt des Trocknens durch Zerstäuben der pulverisierten Mischung mit heißer Luft bei einer Temperatur von 100°C bis 250°C, vorzugsweise von 115°C bis 150°C, insbesondere in einem Zerstäubungsturm, um ein teilweise getrocknetes und nicht-stabilisiertes hygroskopisches Pulver zu erhalten, und einer primären Stabilisation des teilweise getrockneten und nicht-stabilisierten hygroskopischen Pulvers, um eine Zusammensetzung des hygroskopischen Produkts in der Form eines nicht-hygroskopischen stabilisierten Pulvers zu erhalten,
- einen Schritt der sekundären Stabilisation des nicht-hygroskopischen stabilisierten Pulvers, das im vorhergehenden Schritt erhalten wird.

20. Herstellungsverfahren nach einem der Ansprüche 1 bis 19, **dadurch gekennzeichnet, dass** das anfänglich hygroskopische Produkt Lactulose ist, gegebenenfalls in Mischung mit Produkten, die Ernährungs- und/oder therapeutische Eigenschaften aufweisen.

## Claims

1. Process for the preparation of a non-hygroscopic pulverulent composition, comprising a stage of spray drying, without an atomization support, of an aqueous solution containing at least one originally hygroscopic product, and a cryogenic fluid, in particular a food-quality cryogenic fluid, or a mixture of cryogenic fluids, in particular chosen from carbon dioxide, nitrogen, liquid air, said aqueous solution being obtained by dissolving said cryogenic fluid in an initial aqueous solution containing said originally hygroscopic product, **characterized in that** the originally hygroscopic product is lactulose.

2. Preparation process according to claim 1, **characterized in that** the stage of spray drying is accompanied by a primary stabilization resulting from the cooling of the pulverulent composition obtained during the stage of spray drying.

3. Preparation process according to claim 2, **characterized in that** the cooling of the pulverulent composition obtained during the spray drying stage, takes place within a temperature range lower than the glass transition temperature of the hygroscopic product, and **in that** the water content of said pulverulent composition obtained on completion of said stage is less than 7%, and is in particular from 1% to 4%.

4. Preparation process according to any one of claims 1 to 3, **characterized in that** the spray drying stage is preceded by a stage of simultaneous spraying of an anti-agglomeration agent and the aqueous solution containing lactulose, and food-quality cryogenic fluid.

5. Preparation process according to claim 4, **characterized in that** the concentration of anti-agglomeration agent is less than 0.5%, and varies preferably from 0.1% to 0.3% by weight of dry extract of the anti-agglomeration agent relative to the weight of dry extract of the non-hygroscopic pulverulent composition of lactulose.

6. Preparation process according to claim 4 or 5, **characterized in that** the anti-agglomeration agent is chosen from: colloidal silica, silicates, magnesium carbonate, calcium, talc and phosphate.

7. Preparation process according to any one of claims 1 to 6, **characterized in that** the stage of spray drying is carried out with hot air at a temperature of 100°C to 250°C, preferably of 115°C to 150°C.

8. Preparation process according to any one of claims 1 to 7, **characterized in that** the initial aqueous solution containing lactulose, is at a temperature of 50°C to 85°C, preferably of 65°C to 80°C.

9. Preparation process according to any one of claims 1 to 8, **characterized in that** the initial aqueous solution containing the originally hygroscopic product, lactulose, has a dry matter concentration of 20% to 80% by weight of dry matter relative to the weight of the initial aqueous solution, and preferably of 60% to 70%.

10. Preparation process according to any one of claims 1 to 9, **characterized in that** the initial aqueous solution containing lactulose, contains 20% to 100%, in particular 50% to 100%, and preferably 60% to 80% by weight of hygroscopic product relative to the total weight of dry matter.

11. Preparation process according to any one of claims 1 to 10, **characterized in that** the cryogenic fluid is at a pressure comprised of 10⁵ Pa to 20 x 10⁵ Pa, preferably of 4 x 10⁵ Pa to 12 x 10⁵ Pa.

12. Preparation process according to any one of claims 1 to 11, **characterized in that** the aqueous solution containing lactulose and the cryogenic fluid, is sprayed at a pressure of 2 x 10⁶ Pa to 2 x 10⁷ Pa.

13. Preparation process according to any of claims 1 to 12, **characterized in that** the stage of spray drying and primary stabilization resulting in a sprayed mixture is followed by a stage of secondary stabilization by cooling said sprayed mixture by secondary air which is partially dehydrated.

14. Process according to any one of claims 1 to 13, **characterized in that** the non-hygroscopic pulverulent composition of lactulose, obtained at the outlet of the stage of spray drying and primary stabilization and the stage of secondary stabilization, carried out in a spraying tower, is introduced into one or more cyclone(s).

15. Process according to any one of claims 1 to 14, **characterized in that** the non-hygroscopic pulverulent composition of lactulose is recovered at the base of the spraying tower, in microgranulated powder form, the average grain size of which can vary from 100 µm to 500 µm.

16. Process according to any one of claims 1 to 14, **characterized in that** the non-hygroscopic pulverulent composition of lactulose is recovered at the outlet of one of the cyclones.

17. Process according to any one of claims 1 to 16, **characterized in that** the aqueous solution containing lactulose, and a cryogenic fluid is co-dried by spraying with a hygroscopic substance in powder form.

18. Process according to claim 17, **characterized in that** the substance in hygroscopic powder form is chosen from organic products the average molecular mass of which is less than 1,000 Da, in particular comprising at least 50% by weight of glucides, such as lactulose, fructo-oligosaccharides, fructose, saccharose, glucose or mixtures of these, polyols such as sorbitol, maltitol or xylitol, honey-based compositions, products derived from the extraction of lactose, lactoserum or its derivatives, mixtures of sugars and sweeteners, such as mixtures of fructo-oligosaccharides and aspartame, acesulphame or rhamnose.

19. Continuous preparation process of a non-hygroscopic pulverulent composition according to any one of claims 1 to 18, said process being **characterized in that** it comprises the following stages:
- a stage of heating an initial aqueous solution containing an originally hygroscopic product, lactulose having a glass transition temperature of 10°C to 110°C, at a temperature of 50°C to 85°C, preferably at 65°C to 80°C, in order to obtain a heated initial aqueous solution,
- a stage of dissolution of a cryogenic fluid, in particular of a food-quality cryogenic fluid or a mixture of cryogenic fluids, in said heated initial aqueous solution, said cryogenic fluid being in particular chosen from carbon dioxide, nitrogen, liquid air, and being at a pressure comprised of 10⁵ Pa to 20 x 10⁵ Pa, preferably of 4 x 10⁵ Pa to 12 x 10⁵ Pa, in order to obtain an aqueous solution containing said hygroscopic product and a cryogenic fluid,
- a stage of simultaneous spraying of an anti-agglomeration agent and the aqueous solution containing said hygroscopic product and a cryogenic fluid, in order to obtain a sprayed mixture,
- a stage of spray drying of said sprayed mixture with hot air at a temperature of 100°C to 250°C, preferably of 115°C to 150°C, in particular in a spraying tower, in order to obtain a hygroscopic powder, partially dried and not stabilized, and primary stabilization of said partially dried and not stabilized hygroscopic powder, in order to obtain a composition of said hygroscopic product in the form of a non-hygroscopic stabilized powder,
- a stage of secondary stabilization of the non-hygroscopic stabilized powder obtained in the previous stage.

20. Preparation process according to one of claims 1 to 19, **characterized in that** the originally hygroscopic product is lactulose, optionally in a mixture with products having nutritional and/or therapeutic properties.
